# EUROPEAN PATENT APPLICATION

(11) **EP 4 473 996 A1**
(43) Date of publication of application: **11.12.2024**
(21) Application number: 23749672.4
(22) Date of filing: 26.01.2023
(51) Int. Cl.: A61M 25/09, A61M 25/01, A61M 25/095

(54) **DISTAL STABILIZER AND METHOD FOR IMAGING DISTAL STABILIZER**

(30) Priority: 02.02.2022 JP 2022015186; 06.06.2022 JP 2022091404; 27.07.2022 JP 2022119164
(71) Applicant: Bolt Medical Inc., Tokyo 103-0023 (JP)
(72) Inventor: INUZUKA Naoki, Tokyo 103-0023 (JP)
(74) Representative: Ström & Gulliksson AB
(86) International application number: PCT/JP2023/002524
(87) International publication number: WO 2023/149356

(57) **Abstract**

Provided is a distal stabilizer capable of more securely and more quickly identifying slack of a delivery wire or a microcatheter and aberrant of the same into a mass. The distal stabilizer 1, which is to be used for delivering a catheter in a biological lumen, comprises: a linear delivery member 3; an anchor device 2 extending from the distal end of the linear delivery member 3 and capable of being engaged to the inner wall of the biological lumen; and a visible part 4 provided on the distal side of the linear delivery member 3 and including a radiopaque part that is bendable in the axial direction together with the linear delivery member 3.

## Description

### TECHNICAL FIELD

The present invention relates to a distal stabilizer for use in a catheter delivery in a biological lumen, and a method for imaging the distal stabilizer.

### BACKGROUND ART

Treatment by a treatment device or treatment using a catheter itself as the treatment device has been performed by using a pathway inside the catheter of which a distal end has been guided to the vicinity of a target position, and delivering the treatment device to the target position therethrough in a biological lumen of a patient. For example, Patent Document 1 discloses a distal stabilizer (anchor device) in which a stent for anchoring is joined to the distal end of a delivery wire. When this stent is released from a microcatheter and expanded, since the stent is anchored to the blood vessel inner wall, it is possible to easily perform an operation of delivering a treatment catheter that is placed over the microcatheter to the vicinity of a target position.

### Citation List

### Patent Document

Patent Document 1: US Patent No. 968221

### DISCLOSURE OF THE INVENTION

### Problems to be Solved by the Invention

Various treatments using a treatment device have been carried out to an affected site within the biological lumen. As tools for performing the operation of arranging such a treatment device in the vicinity of the target position in the biological lumen, the aforementioned treatment catheter, one or a plurality of microcatheters which are thinner than the treatment catheter, and a delivery wire placed in this microcatheter in an inserted manner are commonly used. As typical operation is performed by placing the delivery wire at the distal side in a prior manner, guiding the microcatheter mounted around this delivery wire to the vicinity of the target position, and then guiding the treatment catheter mounted around the microcatheter to the vicinity of the target position.

In a case in which a lump is present within a blood vessel which is a biological lumen, when the delivery wire or the microcatheter is misplaced into such a lump, the treatment catheter having a larger inner diameter than the microcatheter loses its advanced capability in the intended direction, resulting in poor delivery performance. In particular, the delivery performance of the treatment catheter is significantly reduced at a lump that is present in extremely tortuous portions of the vessel. With a conventional distal stabilizer, in an X-ray transmission image, it is difficult for a practitioner to understand that such a delivery wire or microcatheter has been misplaced into a lump in an X-ray transmission image, and there has been a problem in that effort and time are required to deliver the treatment catheter to the vicinity of a lump within a blood vessel, or the vicinity of a target position which is closer to the distal side than the lump.

In addition, the delivery wire enters a relaxed state or enters a state under tension within a blood vessel by the pushing or pulling by the practitioner. When the delivery wire within the blood vessel greatly relaxes, problems may arise such as the delivery wire being misplaced into a lump formed within the blood vessel or a different blood vessel from the target position, and delivery along the true lumen not being possible upon delivering the treatment catheter to the distal side. In addition, the risk raises of damaging the blood vessel by the delivery wire or the microcatheter placed over the delivery wire, or problems may also arise such as damage to the treatment device, by forcible pushing of the delivery wire. On the other hand, when a strong tensile force acts on the delivery wire within a blood vessel, problems may arise such as excessive tensile load acting on the blood vessel itself by the locked stent, and sliding and displacement of the stent locked against the blood vessel inner wall occurring. Conventionally, the practitioners are required to operate the deliver wire as a skill based on individual knowledge and experience. Therefore, it has not been recognized as an issue to configure to appropriately operate the delivery wire by referring to visual information to enable safer procedures, not just based on individual knowledge and experience.

The present invention has a first object of providing a distal stabilizer that makes it possible to more reliably and more quickly understand the relaxing of the delivery wire or the microcatheter and the misplacement of the delivery wire or the microcatheter into a lump. In addition, the present invention has a second object of providing a distal stabilizer and a method of imaging the distal stabilizer that enable safer procedures in various treatments to affected sites.

### Means for Solving the Problems

A first aspect of the present invention relates to a distal stabilizer for use in catheter delivery within a biological lumen, and the distal stabilizer includes: a linear delivery member; an anchor device extending from a distal end of the linear delivery member and capable of being locked against an inner wall of the biological lumen; and a visible part that is provided on a distal side of the linear delivery member and includes an X-ray opaque portion that is bendable in an axial direction together with the linear delivery member.

According to a second aspect of the present invention, in the distal stabilizer as described in the first aspect, the visible part is provided along the axial direction from the distal end toward a proximal side of the linear delivery member.

According to a third aspect of the present invention, in the distal stabilizer as described in the first or second aspect, the distal stabilizer is used in a biological lumen having a ratio of an estimated neck length of a lump formed within the biological lumen and a length in an axial direction of the visible part satisfying 1:1 to 1:20.

According to a fourth aspect of the present invention, in the distal stabilizer as described in the first or second aspect, the length in the axial direction of the visible part is 10 mm to 500 mm.

According to a fifth aspect of the present invention, in the distal stabilizer as described in any one of the first to fourth aspects, the X-ray opaque portion is provided on 10% or more of a surface area of the visible part.

According to a sixth aspect of the present invention, in the distal stabilizer as described in any one of the first to fifth aspects, the X-ray opaque portion is configured by a tubular member provided on an outer circumferential surface of the linear delivery member.

According to a seventh aspect of the present invention, in the distal stabilizer as described in any one of the first to fifth aspects, the X-ray opaque portion is configured by a wire-shaped member wound around the outer circumferential surface of the linear delivery member.

According to an eighth aspect of the present invention, in the distal stabilizer as described in any one of the first to seventh aspects, the X-ray opaque portion is provided continuously or discontinuously in an axial direction of the visible part.

A ninth aspect of the present invention relates to a method of imaging the distal stabilizer according to any of the first aspect to the eighth aspect within a biological lumen, and the method includes: a first step of imaging the visible part of the distal stabilizer which is sent into a biological lumen together with the linear delivery member, prior to an operation of pushing or pulling the linear delivery member; and a second step of imaging the visible part, after the operation of pushing or pulling the linear delivery member.

A tenth aspect of the present invention may be a method of imaging the distal stabilizer according to any of the first aspect to the eighth aspect within a biological lumen, and the method includes: a first step of imaging the visible part of the distal stabilizer sent together with the linear delivery member within a biological lumen; a second step of imaging the visible part during an operation of pushing or pulling the linear delivery member; and a third step of imaging the visible part after the operation of pushing or pulling the linear delivery member, in which the operations of imaging from the first step to third step are performed continuously or discontinuously.

### Effects of the Invention

According to the present invention, it is possible to provide a distal stabilizer that makes it possible to more reliably and more quickly understand the relaxing of the delivery wire or the microcatheter and the misplacement of the delivery wire or the microcatheter into a lump. In addition, according to the present invention, it is possible to provide a distal stabilizer and a method of imaging the distal stabilizer that enable safer procedures in various treatments to affected sites.

### BRIEF DESCRIPTION OF THE DRAWINGS

FIG. 1 is a view showing a delivery system 10 including a distal stabilizer 1 according to a first embodiment;
FIG. 2A is a view for explaining other configurations of a visible part 4;
FIG. 2B is a view for explaining other configurations of the visible part 4;
FIG. 3 is a view for explaining the distal stabilizer 1 in which a delivery wire 3 is misplaced into aneurysm AN within biological lumen V;
FIG. 4 is a view for explaining the distal stabilizer 1 in which the delivery wire 3 is placed back to the biological lumen V from the aneurysm AN;
FIG. 5 is a view for explaining the distal stabilizer 1 in a state in which the delivery wire 3 is relaxed within the biological lumen V;
FIG. 6 is a view for explaining the distal stabilizer 1 in a state in which excessive tensile load is applied to the delivery wire 3 within the biological lumen V;
FIG. 7 is a view for explaining the distal stabilizer 1 in which the delivery wire 3 is returned to a state with no slack within the biological lumen V;
FIG. 8 is a photographic image showing the distal stabilizer 1 in a state in which the delivery wire 3 is relaxed within the biological lumen V;
FIG. 9 is a photographic image showing the distal stabilizer 1 in which the delivery wire 3 is returned to a state with no slack within the biological lumen V;
FIG. 10A is a view for explaining the configuration of a modified embodiment of the visible part 4;
FIG. 10B is a view for explaining the configuration of a modified embodiment of the visible part 4; and
FIG. 10C is a view for explaining the configuration of a modified embodiment of the visible part 4.

### PREFERRED MODE FOR CARRYING OUT THE INVENTION

Hereinafter, embodiments of a distal stabilizer according to the present invention will be explained. It should be noted that the drawings attached to the present specification are all schematic diagrams, and the shape, scale and dimensional ratios of vertical and horizontal of each part have been changed or exaggerated from the actual product in consideration of ease of understanding, etc. For example, the drawings are illustrated in such a manner that the longitudinal direction of the catheter, etc., is shorter, and the radial direction is thicker. In the present specification, etc., the terminology specifying the shape, geometric conditions, and extents of these, e.g., a term such as "direction" encompasses not only the direction according to the strict meanings of such a term but also directions that can be considered as substantially or roughly the same direction. In addition, in the present specification, the long axis direction in a state stretching the distal stabilizer 1 in a straight line is referred to as "axial direction LD". Further, in the axial direction LD, a proximal side close to the practitioner is explained as "D1", and a distal side away from the practitioner is explained as "D2".

### (First Embodiment)

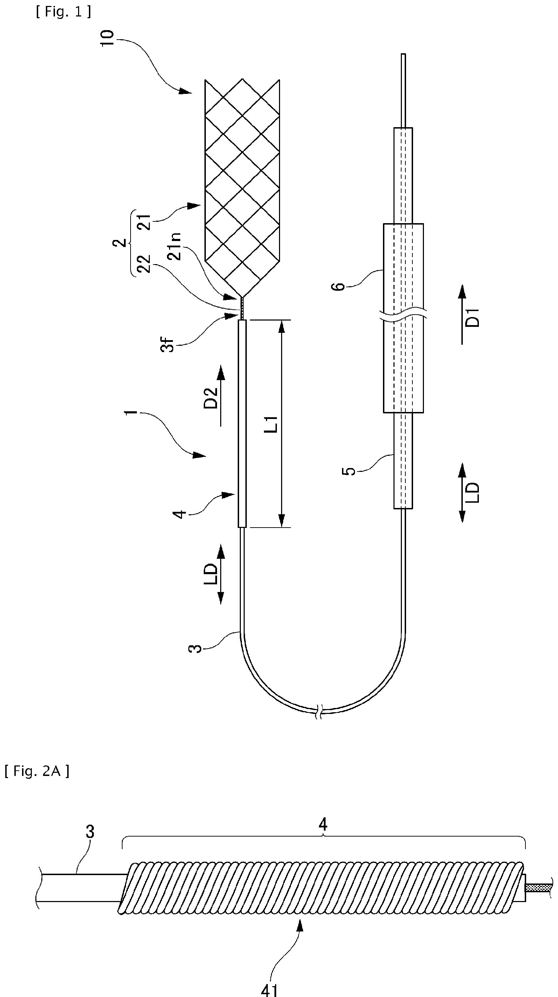

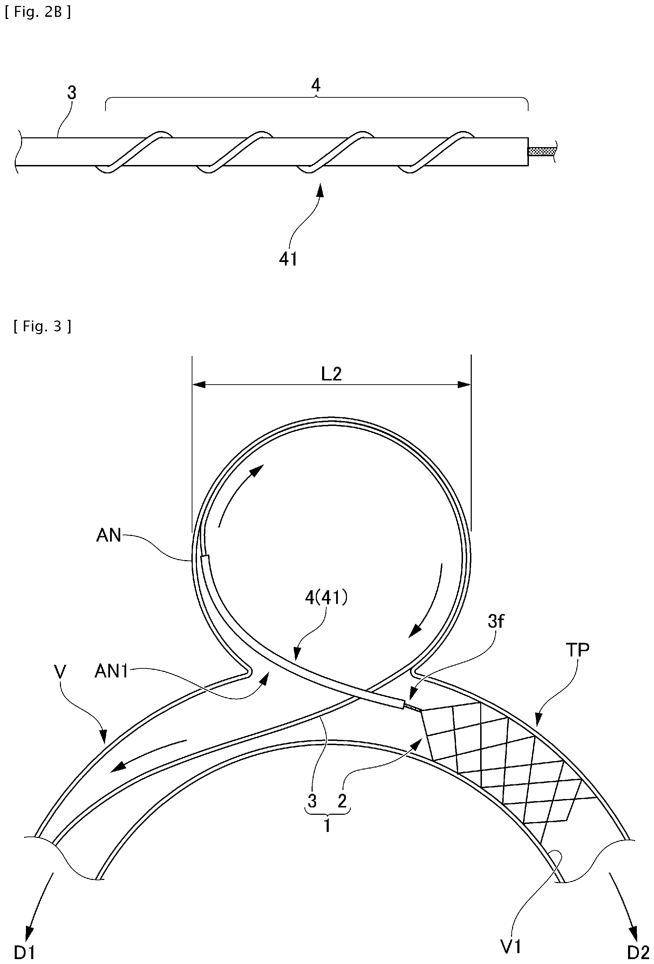

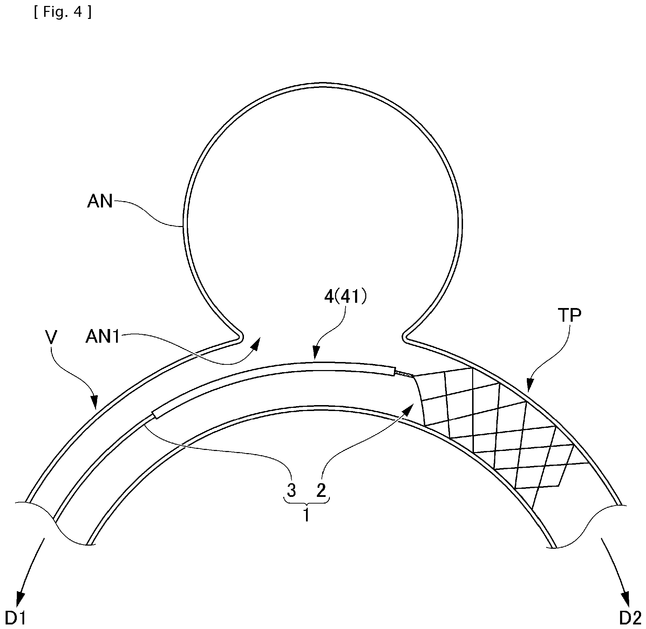

FIG. 1 is a view showing a delivery system 10 including a distal stabilizer 1 according to a first embodiment. FIG. 2A and FIG. 2B are views for explaining other configurations of a visible part 4. FIG. 3 is a view for explaining the distal stabilizer 1 in which a delivery wire 3 is misplaced into aneurysm AN within biological lumen V. FIG. 4 is a view for explaining the distal stabilizer 1 in which the delivery wire 3 is placed back from the aneurysm AN to the biological lumen V.

The delivery system 10 shown in FIG. 1 is a system for use in the delivery of a treatment device in a biological lumen. A blood vessel which is a biological lumen in which the delivery system 10 is used not particularly limited. However, typically, a highly tortuous meandering cerebral blood vessel or the like can be exemplified. In the present embodiment, an example using the delivery system 10 in a cerebral blood vessel in which an aneurysm exists will be explained.

As shown in FIG. 1, the delivery system 10 includes the distal stabilizer 1, and a plurality of catheters including a first catheter 5 and a second catheter 6. The distal stabilizer 1 is a device for use in catheter delivery in a biological lumen. The distal stabilizer 1 includes a locking stent 2, and a delivery wire (linear deliver member) 3.

The locking stent 2 is an anchor device that extends from a distal end 3f of the delivery wire 3 and can be locked against an inner wall V1 (refer to FIG. 3) of the biological lumen V by its self-expansion force. It should be noted that the biological lumen is not particularly limited, and may be cerebral, coronary, upper and lower limb blood vessels (arteries, veins), organs, etc. The locking stent 2 includes a main body 21 and an antenna 22. The main body 21, for example, is a mesh-like structure, and is placed in the first catheter 5 in an inserted manner and in a reduced-diameter state. FIG. 1 shows a state in which the main body 21 is expanded. The antenna 22 is a part that focuses a proximal end 21n of the main body 21 onto the delivery wire 3. Although not illustrated, the locking stent 2 includes a marker made of an X-ray opaque material on each of the distal end and the proximal end. The markers of the locking stent 2 are indicators for confirming the positions of the distal end and proximal end of the locking stent 2 in an X-ray transmission image, and differs from a visible part 4 (described later) provided on the delivery wire 3.

The delivery wire 3 is a member used for advancing and retracting the locking stent 2 within the biological lumen. The delivery wire 3 is sent out to the distal side D2 upon advancing the locking stent 2 within the biological lumen V, and is drawn in to the proximal side D1 upon retracting the locking stent 2 within the biological lumen V. The delivery wire 3, for example, is made of a material having high elastic modulus such as stainless steel. In addition, the diameter of the delivery wire 3 is not particularly limited so long as having sufficient physical properties to perform the operations of advancing and retreating within the biological lumen V, and conforming to the first catheter 5, and may be 0.005 to 0.018 inches, for example.

The delivery wire 3 includes the visible part 4. The visible part 4 is provided along the axial direction from the distal end 3f toward the proximal side D1 of the delivery wire 3. The visible part 4 is a structure including an X-ray opaque portion 41 (described later) which is bendable in the axial direction LD together with the delivery wire 3.

The X-ray opaque portion 41 is a portion having high X-ray opacity. For this reason, the X-ray opaque portion 41 has high visibility compared to other portions in the X-ray transmission image obtained by irradiation of X-rays. As described later, the X-ray opaque portion 41 serves as an indicator for visually recognizing that a part of the delivery wire 3 is misplaced into the aneurysm AN in the X-ray transmission image. Examples of the X-ray opaque materials forming the X-ray opaque portion 41 include platinum, gold, tantalum, platinum, tungsten, iridium, platinum tungsten, etc., and alloy materials of these can be exemplified. Examples of the alloy material include a polymer material having X-ray opaqueness to which an X-ray opaque filler or the like is added.

In the first embodiment and a second embodiment described later, the X-ray transmission image is photographed by an X-ray imaging device (not shown). The X-ray imaging device is a device which photographs an X-ray transmission image as a moving image or a still image. The practitioner can visually recognize in real-time the behavior of the delivery wire 3 and the visible part 4 within the biological lumen, by projecting an X-ray transmission moving image captured by the X-ray imaging device onto a monitor screen. In addition, the practitioner can visually recognize the state of the delivery wire 3 and the visible part 4 within the biological lumen by projecting an X-ray transmission still image photographed by the X-ray imaging device onto the monitor screen.

It is desirable for the X-ray opaque portion 41 to be provided on 10% or more of the surface area of the visible part 4. In the visible part 4 of the first embodiment (and the second embodiment described later), the X-ray opaque portion 41 is configured by a tubular member covering the outer circumferential surface of the delivery wire 3. The X-ray opaque portion 41 configured by the tubular member is continuous in the axial direction, and covers the entirety of the surface area of the visible part 4. In other words, in the configuration of the first embodiment, the X-ray opaque portion 41 is formed on 100% of the surface area of the visible part 4. The visible part 4 of the present embodiment can be fabricated, for example, by providing the tubular X-ray opaque portion 41 over the delivery wire 3. It should be noted that the configuration in which the X-ray opaque portion 41 is formed on 100% of the surface area of the visible part 4 is an example, and the present invention is not limited to this example. In addition, as described later, the X-ray opaque portion 41 may be discontinuous in the axial direction of the visible part 4.

The outer diameter of the visible part 4 is not particularly limited so long as conforming to the first catheter 5, and may be 0.1 to 1 mm, for example. In addition, the length L1 in the axial direction of the visible part 4 depends on the biological lumen in which the delivery system 10 is used; however, in an application delivering a treatment catheter to the vicinity of a target position TP for treatment of an aneurysm AN described later, the length L1 is 30 to 100 mm, for example. The length L1 of the visible part 4 is not particularly limited; however, as shown in FIG. 3, for example, the length L1 may be at least an estimated neck length L2 of the aneurysm AN. As described later, when the length L1 of the visible part 4 is at least the estimated neck length L2 of the aneurysm AN, even if the visible part 4 is misplaced into the aneurysm AN from any portion, the practitioner can visually recognize that a part of the delivery wire 3 is misplaced into the aneurysm AN in the X-ray transmission image. In such a usage mode, the distal stabilizer 1, for example, is suitably used in a biological lumen in which the ratio of the estimated neck length L2 of the aneurysm AN to the length L1 in the axial direction of the visible part 4 satisfies 1:1 to 1:20. In addition, the length L1 in the axial direction of the visible part 4 is 10 to 500 mm, for example, in an application delivering the treatment catheter to the vicinity of the target position TP, which is located on the distal side within a bent blood vessel, as in the second embodiment (described later). It should be noted that the relationship between an application of the distal stabilizer 1 and the length L1 in the axial direction of the visible part 4 in such an application is not limited to the above-mentioned example, and can be selected as appropriate. The length L1 in the axial direction of the visible part 4 is more efficient as it is longer, and may be a length which is 100% relative to the length in the axial direction of the delivery wire 3, for example.

The X-ray opaque portion 41 of the visible part 4 is not limited to the tubular shape shown in FIG. 1, and may include another configuration. FIG. 2A and FIG. 2B each show an example configuring the X-ray opaque portion 41 of the visible part 4 by a wire-shaped member. In the visible part 4 shown in FIG. 2A, an X-ray opaque material formed in a wire shape is wound without any gap on the delivery wire 3. In the visible part 4 shown in FIG. 2B, an X-ray opaque material formed in a wire shape is wound so that gaps are provided on the delivery wire 3. It should be noted that, as described later, the visible part 4 is not limited to the example of the embodiment, and can be configured in various forms.

As shown in FIG. 1, the first catheter 5 is placed over the delivery wire 3. The first catheter 5, for example, is a catheter called a microcatheter. A second catheter 6 (described later) is placed over the first catheter 5. The diameter of the first catheter 5 is set according to the inner diameter and the bending degree of the biological lumen V at the target position TP and the path leading thereto, and thus is not particularly limited; however, the inner diameter is preferably 0.017 inches or less, and more preferably 0.0165 inches or less. One or a plurality of catheters (not shown) placed over the second catheter 6 may be used as necessary. Generally, by using many catheters, it is ultimately possible to insert and advance a catheter having a large inner diameter into the biological lumen V.

Among the plurality of catheters including the second catheter 6, a catheter having a larger inner diameter than the first catheter 5 is called a treatment catheter. The treatment catheter is a catheter having an inner diameter sufficient for the insertion of the treatment device, or an inner diameter sufficient to use the catheter itself as a treatment device. The treatment catheter may also be called a guiding catheter in the application for inserting the treatment device. Examples of the treatment device include thrombus aspiration devices, flow diverters, aneurysm embolization devices, thrombus removal devices (stent retrievers, etc.), stents for treating aneurysms, stents for treating intracranial artery stenosis, balloon catheters, shunts, and liquid embolic material release means (catheters including lumens that allow liquid embolic material to pass therethrough). The treatment catheter itself may be used as a treatment device. In such an application, the treatment catheter also may be called a thrombus aspiration catheter. In the embodiment described later, a case of the second catheter 6 being the treatment catheter is explained as an example.

Next, the sequence of part of the procedure for delivering the treatment device to the target position TP using the delivery system 10 of the first embodiment will be explained. It should be noted that, as the procedure, various operations other than the following description are carried out; however, explanation for them will be omitted herein. The biological lumen V is a blood vessel. In particular, the distal stabilizer 1 and the delivery system 10 of the present embodiment are suitably used in a case of highly bent and meandering blood vessels being included in the blood vessel. In addition, the distal stabilizer 1 and delivery system 10 of the present embodiment are preferably used in a case of the target position TP being located in a region no more than 7 mm in blood vessel inner diameter, more specifically, less than 2.5 mm (preferably no more than 2.0 mm or no more than 1.5 mm). More specifically, the target position TP may be regions (M2, M3, M4, etc.) including and after M2 of the middle cerebral artery (MCA), A1 and A2 regions of anterior cerebral artery (ACA), regions (P1, P2, etc.) including and after P1 of the posterior cerebral artery (PCA), internal artery carotid (ICA), etc. However, the target position TP is not limited thereto, and may be located in a region of a wide range of 0.5 to 10 mm in blood vessel inner diameter.

In the delivery system 10, the treatment device is delivered to the vicinity of the target position TP in a state placed in the second catheter (treatment catheter) 6 in an inserted manner. In a case of delivering the treatment device to the target position TP, first, the second catheter 6 is placed on the proximal side D1 of the biological lumen V (not shown). The second catheter having a large diameter has its distal end, and the distal end is typically caught in a bent portion or branched part of the biological lumen V, and is difficult to further advance to the distal side. In particular, in highly bent and meandering blood vessels, this trend is remarkable. The first catheter 5 is inserted into the second catheter 6 which has hardly advanced, and is sent into the biological lumen V. Then, the first catheter 5 is pushed out from the distal end of the second catheter 6, and the distal end of the first catheter 5 is disposed in the vicinity of the target position TP.

Next, the distal stabilizer 1 (refer to FIG. 1) is inserted into the first catheter 5, and disposed in the vicinity of the target position TP (not shown). At this time, the locking stent 2 of the distal stabilizer 1 is housed in the first catheter 5 in the reduced-diameter state. Next, the locking stent 2 housed in the first catheter 5 is released from the distal end of the first catheter 5. The release of the locking stent 2 is performed by an operation of retracting the first catheter 5 to the proximal side D1. The locking stent 2 released from the distal end of the first catheter 5 expands on its own by its self-expansion force. This self-expansion force acts as a force pressing the inner wall V1 of the biological lumen V from the inside toward the outside. Therefore, the locking stent 2 is locked against the inner wall V1, as shown in FIG. 3. It should be noted that, for the purpose of easy understanding of the shape of the visible part 4, FIG. 3 shows a state in which the first catheter 5 is retracted more toward the proximal side D1 than the visible part 4. However, the retracted position of the first catheter 5 may be a position where the locking stent 2 is released (a position where the visible part 4 is covered).

When the distal end of the first catheter 5 advances toward the target position TP, the first catheter 5 may be misplaced into the aneurysm AN. Since the first catheter 5 has flexibility, the first catheter 5 may advance along the inner wall of the aneurysm AN, and rotate once therein to be placed back again to the biological lumen V. On the other hand, the second catheter 6 which has a larger diameter than the first catheter 5 tends to be caught on the inner wall of the aneurysm AN. Therefore, even if the second catheter 6 is placed over the first catheter 5, the ability of advance to the intended direction is lost, and it becomes difficult for the second catheter 6 to be placed back again to the biological lumen V from the inside of the aneurysm AN. In addition, the second catheter 6 having a large diameter has high rigidity (hardness) compared to a catheter having a small diameter such as the first catheter 5. Therefore, in a case where the second catheter 6 is misplaced into the aneurysm AN, the second catheter 6 may break through the inner wall of the aneurysm AN. For this reason, it is required to avoid that the second catheter 6 having a large diameter is misplaced into the aneurysm AN. Incidentally, in conventional distal stabilizers, since the X-ray opaque markers are only provided at the distal end and the proximal end of the locking stent 2, it is not possible to visually recognize the misplacement of the delivery wire to an aneurysm in an X-ray transmission image.

In contrast, when the first catheter 5 (not shown) is misplaced into the aneurysm AN and the delivery wire 3 inserted in this first catheter 5 is misplaced into the aneurysm AN, the visible part 4 is bent in the axial direction together with the delivery wire 3, and the shape in the axial direction changes. For this reason, the practitioner can more reliably and more quickly understand that the delivery wire 3 and first catheter 5 are misplaced into the aneurysm AN in the X-ray transmission image. This is because, if the delivery wire 3 and first catheter 5 are not misplaced into the aneurysm AN, the shape in the axial direction of the visible part 4 does not change and follows an opening AN1 of the aneurysm AN. When the situation in which the delivery wire 3 and first catheter 5 are misplaced into the aneurysm AN, the practitioner, for example, performs operations to retract the first catheter 5 and then to pull the delivery wire 3 toward the proximal side D1 while the locking stent 2 is locked against the inner wall V1. Since the path of the delivery wire 3 within the biological lumen V thereby shortens, it is possible to place the delivery wire 3 from the inside of the aneurysm AN back to the biological lumen V, as shown in FIG. 4. In a case in which the target position TP is the aneurysm AN, it is possible to allow the delivery wire 3 to be along with the opening AN1 of the aneurysm AN by appropriately performing the aforementioned pulling operation, and then appropriately performing an advancing operation as necessary. In this state, if the second catheter 6 having a larger inner diameter than the first catheter 5 is insert into the delivery wire 3 and first catheter 5 and advances, since the second catheter 6 follows the opening AN1 of the aneurysm AN, it is possible to simply deliver the second catheter 6 to the vicinity of the target position TP. Thereafter, it is possible to deliver the treatment device inserted into the second catheter 6 to the aneurysm AN.

It should be noted that, even in a case where the first catheter 5 is retracted to the position of the visible part 4, it is possible to place the delivery wire 3 and first catheter 5 from the inside of the aneurysm AN back to the biological lumen V by performing the operation to pull the delivery wire 3 toward the proximal side D1. In this state, when the second catheter 6 having a larger inner diameter than the first catheter 5 is inserted into the delivery wire 3 and advances, it is possible to easily deliver the second catheter 6 to the vicinity of the target position TP.

In addition, although FIG. 2 shows an example of anchoring the locking stent 2 to the vicinity of the aneurysm AN, the target position TP may exist closer to the distal side than the aneurysm AN. Even in this case, the practitioner can more reliably and more quickly understand that the delivery wire 3 is misplaced into the aneurysm AN in the X-ray transmission image. More specifically, by selecting the distal stabilizer 1 having the visible part 4 of the appropriate length according to the position and size of the aneurysm AN within the biological lumen V, it is possible to understand that the delivery wire 3 and first catheter 5 are misplaced into the aneurysm AN in the X-ray transmission image showing the moment that the distal end of the first catheter 5 passes the aneurysm AN. Then, it is possible to place the delivery wire 3 and first catheter 5 from the inside of the aneurysm AN back to the biological lumen V by performing the operation to pull the delivery wire 3 toward the proximal side D1 after anchoring the distal end of the first catheter 5 to the target position TP which is closer to the distal end than the aneurysm AN. It should be noted that, also in this case, by establishing the length L1 of the visible part 4 (refer to FIG. 1) as at least the estimated neck length L2 of the aneurysm AN (L1≤L2), even if the visible part 4 is misplaced from any portion to the aneurysm AN, the practitioner can visibly recognize that a part of the delivery wire 3 is misplaced into the aneurysm AN in the X-ray transmission image.

According to the distal stabilizer 1 of the aforementioned first embodiment, the following such effects are achieved, for example. In the distal stabilizer 1 of the embodiment, when the delivery wire 3 is misplaced into the aneurysm AN, the visible part 4 is bent in the axial direction together with the delivery wire 3, and the shape in the axial direction will change. For this reason, the practitioner can more reliably and more quickly understand that the delivery wire 3 is misplaced into the aneurysm AN in the X-ray transmission image. In addition, since the practitioner can visibly recognize the behavior of the delivery wire 3 in the X-ray transmission image while the practitioner pulls the delivery wire 3 toward the proximal side D1, it is possible to operate the delivery wire 3 at the appropriate speed and amount of force. In this way, according to the delivery system 10 of the present embodiment, it is possible for the practitioner to appropriately operate the delivery wire 3 while referring to the visual information (X-ray transmission image), not only based on knowledge and experience when the practitioner places the delivery wire 3 misplaced in the aneurysm AN back to the biological lumen V. For this reason, it is possible for the practitioner to more safely perform procedures to place the delivery wire 3 misplaced in the aneurysm AN back to the inside of the biological lumen V.

In the distal stabilizer 1 of the first embodiment, the visible part 4 is provided along the axial direction from the distal end 3f of the delivery wire 3 towards the proximal side D1. For this reason, in the X-ray transmission image, it is possible to more reliably understand a change in shape in the axial direction of the visible part 4 provided over the delivery wire 3.

In the distal stabilizer 1 of the first embodiment, by establishing the length L1 of the visible part 4 as at least the estimated neck length L2 of the aneurysm AN, even when the visible part 4 is misplaced into the aneurysm AN from any portion, it is possible for the practitioner to understand that the delivery wire 3 is misplaced into the aneurysm AN in the X-ray transmission image. More specifically, the distal stabilizer 1 of the first embodiment is preferably used in a biological lumen having a ratio of the estimated neck length L2 of the aneurysm AN to the length L1 in the axial direction of the visible part 4 satisfying 1:1 to 1:20.

In a case of using the distal stabilizer 1 of the first embodiment in a procedure of locking the locking stent 2 to the vicinity of the distal side of a lump, by establishing the length in the axial direction of the visible part 4 as 30 to 100 mm, it is possible to suppress a decline in flexibility at the distal side of the delivery wire 3 and allow the visible part 4 to be bent in the axial direction together with the delivery wire 3 when the delivery wire 3 is misplaced in a lump within the biological lumen V.

In the distal stabilizer 1 of the first embodiment, the X-ray opaque portion 41 is provided on 10% or more of the surface area of the visible part 4. For this reason, it is possible for the practitioner to visibly recognize more clearly the change in shape when the visible part 4 is bent in the axial direction together with the delivery wire 3, in the X-ray transmission image.

In the distal stabilizer 1 of the first embodiment, in a case where the X-ray opaque portion 41 of the visible part 4 is configured by a tubular member provided over the outer circumferential surface of the delivery wire 3, it is possible to visually recognize a change in shape more linearly when the visible part 4 is bent in the axial direction together with the delivery wire 3, in the X-ray transmission image. Similar effects can also be obtained in the case of providing the X-ray opaque portion 41 to be continuous in the axial direction of the visible part 4.

In the distal stabilizer 1 of the first embodiment, in a case where the X-ray opaque portion 41 of the visible part 4 is configured by a wire-shaped member wound around the outer circumferential surface of the delivery wire 3, it is possible to maintain good visibility in the X-ray transparency image, while suppressing a decline in flexibility on the distal side of the delivery wire 3. As described later, similar effects can also be obtained in the case of providing the X-ray opaque portion 41 to be discontinuous in the axial direction of the visible part 4.

### (Second Embodiment)

Next, as a second embodiment, an operation of returning the delivery wire 3 relaxed within a biological lumen to an unrelaxed state, and a method of imaging the distal stabilizer will be explained. The distal stabilizer 1 described in the second embodiment is the same as the first embodiment. Therefore, the same reference symbols will be assigned to members which are the same as the first embodiment, and redundant explanations will be omitted.

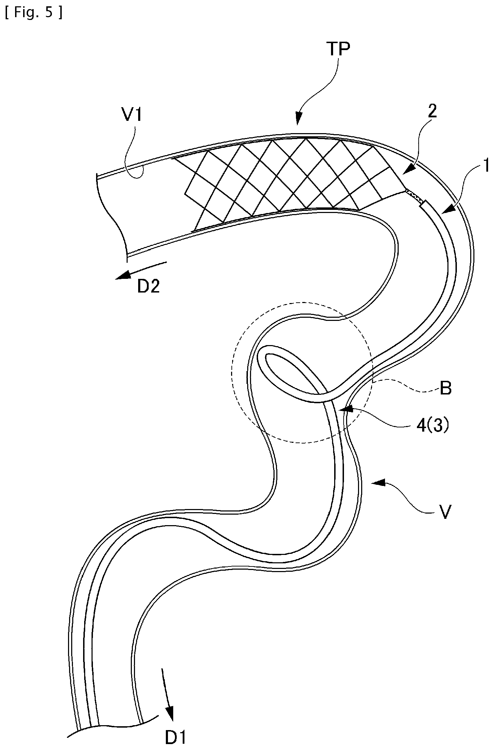

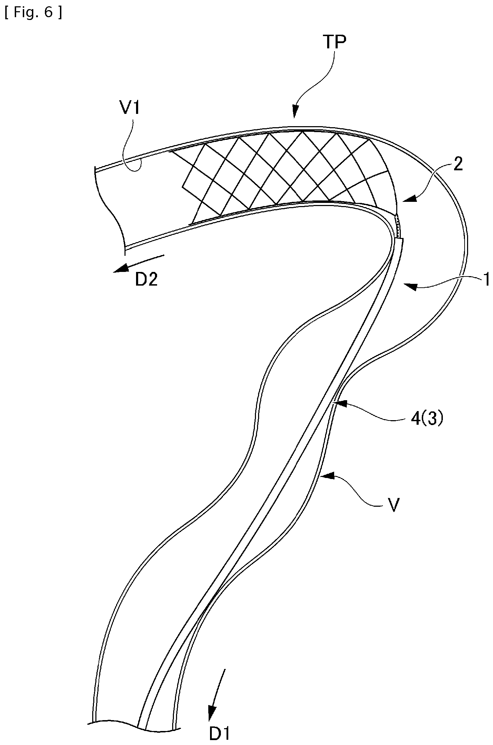

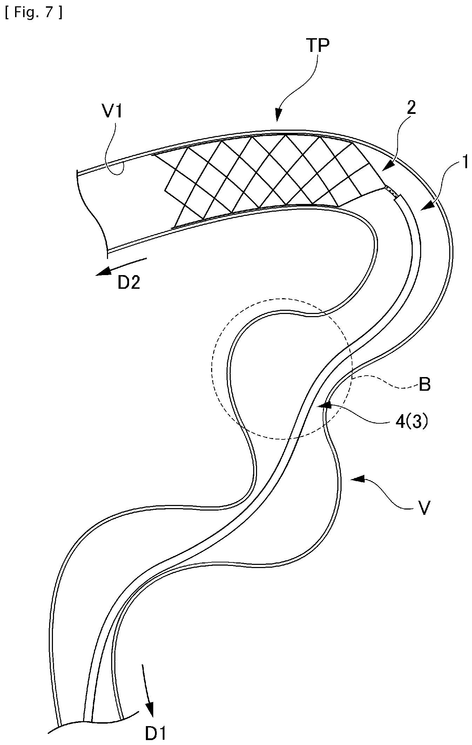

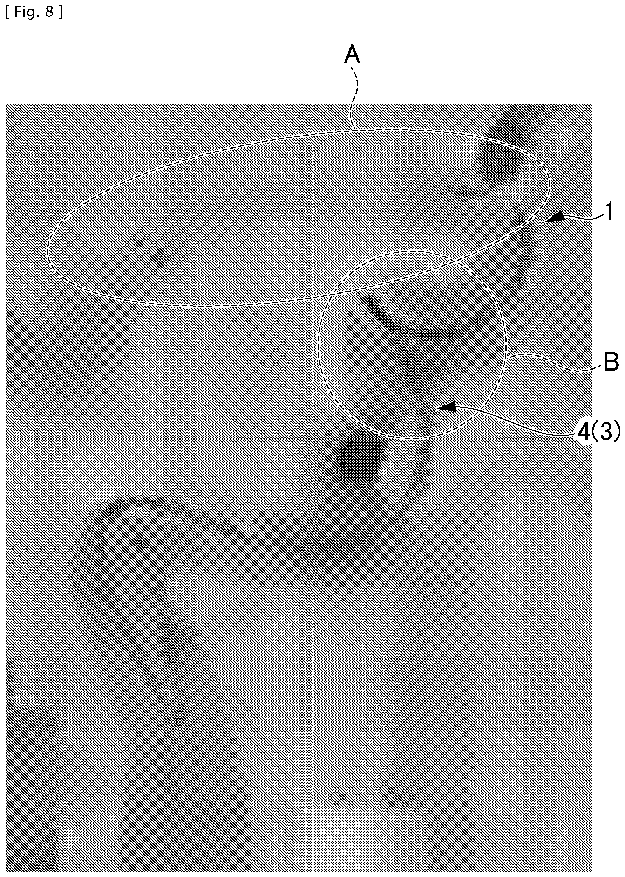

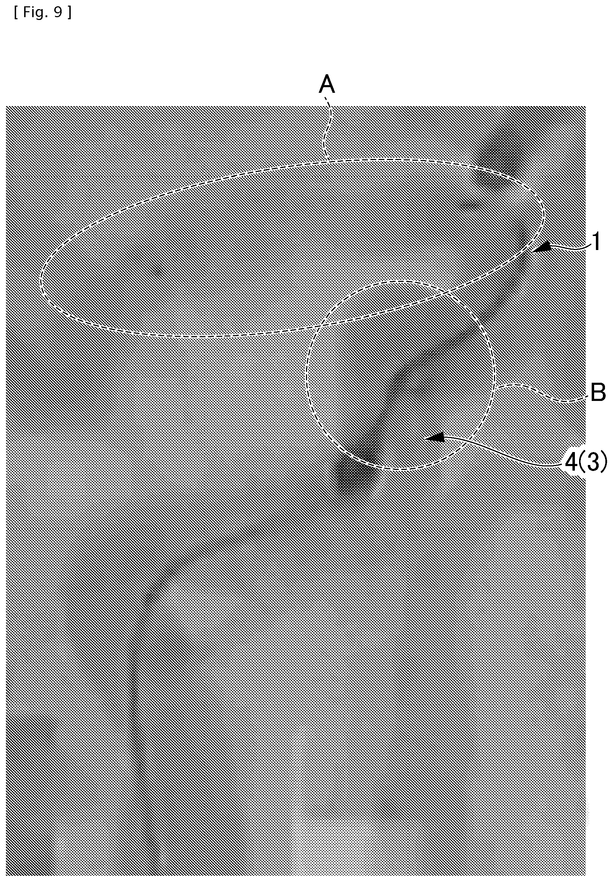

FIG. 5 is a view for explaining the distal stabilizer 1 in which the delivery wire 3 is in a relaxed state within the biological lumen V. FIG. 6 is a view for explaining the distal stabilizer 1 in a state in which excessive tensile load acts on the delivery wire 3 within the biological lumen V. FIG. 7 is a view for explaining the distal stabilizer 1 in which the delivery wire 3 is returned to an unrelaxed state within the biological lumen V. FIG. 8 is a photographic image showing the distal stabilizer 1 in which the delivery wire 3 is a relaxed state within the biological lumen V. FIG. 9 is a photographic image showing the distal stabilizer 1 in which the delivery wire 3 is returned to an unrelaxed state within the biological lumen V. FIG. 8 and FIG. 9 are X-ray transmission images of the distal stabilizer 1 inserted within the biological lumen. FIG. 8 and FIG. 9 each show an image extracting a part of the moving image captured by the X-ray imaging device as a still image. FIGS. 5 to 7 are schematic diagrams created based on the photographic images shown in FIG. 8 and FIG. 9 for facilitating understanding of the second embodiment.

As shown in FIG. 5, the locking stent 2 is locked against the inner wall V1 on the distal side D2 within the biological lumen V. The locking stent 2 does not appear in the X-ray transmission images shown in FIGS. 8 and 9; however, it is locked against the inner wall V1 positioned in a region A shown by the dotted line. Upon pushing the delivery wire 3 to the distal side D2, the delivery wire 3 may greatly relax within the biological lumen V, as shown in FIG. 5 (FIG. 8). In FIG. 5 (FIG. 8), the distal side (which is covered by the visible part 4) of the delivery wire 3 is relaxed to have a ring shape in a meandering region B of the biological lumen V. In this way, when the distal side D2 of the delivery wire 3 greatly relaxes in the biological lumen V, the relaxed portion is misplaced into another blood vessel or the tip portion of the treatment catheter is oriented in an unintended direction upon delivery, which causes problems such as damaging the blood vessel wall. In order to avoid such problems, the practitioner can appropriately operate the delivery wire 3 by referring to an X-ray transmission moving image or still image of the distal stabilizer 1 imaged in the following such sequence.

### (First Imaging Method)

The practitioner images an X-ray transmission moving image of the visible part 4 using an X-ray imaging device (first step). Then, the practitioner delivers the catheter by operating the delivery wire 3 at the appropriate speed and amount of force, while viewing the behavior of the visible part 4 (the delivery wire 3) shown on the monitor screen. When the practitioner delivers the first catheter 5 and second catheter (treatment catheter) 6 using the delivery wire 3, the relaxation is eliminated by pulling the delivery wire 3, and the appropriate route of the catheter is ensured by pushing the wire in a case of excessively pulling the wire, as described later. Even in the middle of such an operation, the imaging of a moving image of the visible part 4 is continuously performed (second step). In addition, the practitioner performs imaging of a moving image of the visible part 4 continuously, even after performing an operation of pushing or pulling the deliver wire 3 while delivering the catheter (third step). The practitioner can push the wire toward the distal side D2 so that the catheter or the delivery wire 3 is not relaxed within the biological lumen V by performing delivery of the catheter and operation of the delivery wire 3 while referring to the X-ray transmission moving image imaged in the aforementioned first to third steps.

### (Second Imaging Method)

The practitioner can perform a second imaging method. The practitioner continuously images an X-ray transmission moving image of the visible part 4 using an X-ray imaging device. Then, the practitioner delivers the catheter by operating the delivery wire 3 at the appropriate speed and amount of force while viewing the behavior of the visible part 4 (the delivery wire 3) shown on the monitor screen. When the practitioner delivers the first catheter 5 and second catheter (treatment catheter) 6 using the delivery wire 3, relaxation is eliminated by pulling the delivery wire 3, and the appropriate route of the catheter is ensured by pushing the wire in a case of excessively pulling the wire, as described later. Before such an operation of pushing or pulling the delivery wire 3, imaging of the image (a moving image or a still image) of the visible part 4 is performed (first step). In addition, imaging of an image of the visible part 4 is also performed after the operation of pushing or pulling the delivery wire 3, (second step). The practitioner can push the wire toward the distal side D2 so that the catheter or the delivery wire 3 is not relaxed within the biological lumen V by performing delivery of the catheter and operation of the delivery wire 3 while referring to the X-ray transmission moving image imaged in the aforementioned first and second steps.

By operating the delivery wire 3 while referring to the X-ray transmission image (a moving image or a still image) imaged with the above-mentioned such sequence, it is possible to avoid the situation in which the relaxed delivery wire 3 is misplaced into a blood vessel different from the target position TP or the tip portion of the catheter is oriented in an unintended direction during delivery of the second catheter (treatment catheter) 6, which causes damaging of the blood vessel walls. In addition to this, it is possible to reduce the risk of damaging the blood vessel by the relaxed delivery wire 3 or the first catheter 5 inserted thereto upon pushing the delivery wire 3 to the distal side D2 and damaging the treatment device due to forcible pushing of the delivery wire 3.

In addition, according to the distal stabilizer 1 of the present embodiment, even when the delivery wire 3 is relaxed on the distal side D2 within the biological lumen V, since the visible part 4 provided on the distal side D2 of the delivery wire 3 is relaxed in the same shape as the delivery wire 3 (not shown), as shown in FIG. 8, it is possible for the practitioner to visually recognize the presence/absence of relaxation of the delivery wire 3 on the distal side D2 more reliably and quickly, and the size and shape thereof, based on the X-ray transmission image (a moving image or a still image) shown on the monitor screen. When the practitioner visually recognizes relaxation of the delivery wire 3 in the X-ray transmission image (a moving image or a still image), in order to eliminate the relaxation, the practitioner performs the operation of pulling the delivery wire 3 toward the proximal side D1 in a state in which the locking stent 2 is locked against the inner wall V1 of the biological lumen V. At this time, the practitioner can operate the delivery wire 3 at the appropriate speed and amount of force, while viewing the behavior of the visible part 4 (the delivery wire 3) shown in the X-ray transmission image (a moving image or a still image). For this reason, it is possible to suppress problems such as excessively pulling the delivery wire 3 in order to eliminate the relaxation of the delivery wire 3 and excessive tensile load acting on the blood vessel itself by the locked locking stent 2, and sliding or dislocation of the locking stent 2 locked within the biological lumen V occurring, as shown in FIG. 6. It should be noted that, as shown in FIG. 6, in a case of excessively pulling the delivery wire 3, it is possible to suppress excessive tensile load acting on the blood vessel itself or the locking stent 2 by pushing the delivery wire 3.

By performing an operation of pulling the delivery wire 3 toward the proximal side D1, since the path of the delivery wire 3 within the biological lumen V becomes short, it is possible to recover the delivery wire 3 relaxed to have a ring shape in the meandering region B of the biological lumen V to an unrelaxed state, as shown in FIG. 7 and FIG. 9. This also applies to other meandering regions, and it is possible to return the relaxed delivery wire 3 to the unrelaxed state. In this way, according to the delivery system 10 of the present embodiment, upon returning the delivery wire 3 relaxed in the biological lumen V to the unrelaxed state, it is possible for the practitioner to perform the appropriate operation by confirming the tensile load acting on the delivery wire 3 on hand while referring to visual information (an X-ray transmission moving image or still image), not only based on knowledge and experience. For this reason, it is possible for the practitioner to more safely perform procedures of pushing the delivery wire 3 to the distal side D2 within the biological lumen V, and returning the delivery wire 3 relaxed within the biological lumen V to an unrelaxed state.

Although embodiments of the present invention have been explained above, the present invention is not to be limited to the aforementioned embodiments, and various modifications and changes thereto are possible as in the modified embodiments described later, and these are also encompassed within the technical scope of the present invention. In addition, the effects described in the embodiment are merely exemplifying the most preferred advantageous effects derived from the present invention, and are not limited to those described in the embodiments. It should be noted that the aforementioned embodiments and modified embodiments described later can be used by combining as appropriate; however, detailed explanations thereof will be omitted.

### (Modified Embodiment)

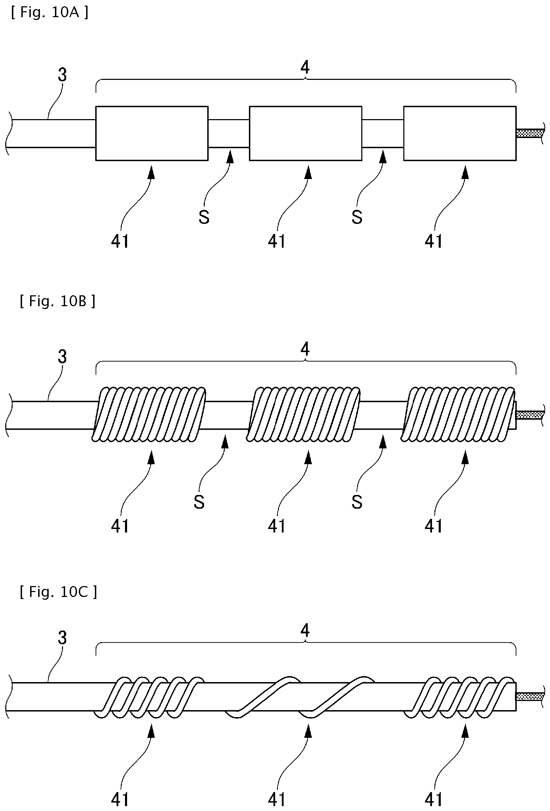

FIGS. 10A to 10C are drawings that each explains the configuration of a modified embodiment of the visible part 4. FIG. 10A shows a modified embodiment of the tubular visible part 4 shown in FIG. 1 of the embodiment. As shown in FIG. 10A, the visible part 4 may include a plurality of tubular X-ray opaque portions 41. In the modified embodiment shown in FIG. 10A, the X-ray opaque portions 41 are provided discontinuously in the axial direction. In the modified embodiment shown in FIG. 10A, gaps S provided between adjacent X-ray opaque portions 41 may be regularly provided or may be irregularly provided. In the modified embodiment shown in FIG. 10A, the length of each of the gaps S, for example, is 1 to 10 mm.

FIG. 10B shows a modified embodiment of the wire-shaped visible part 4 shown in FIG. 2A of the embodiment. As shown in FIG. 10B, the visible part 4 may include a plurality of the wire-shaped X-ray opaque portions 41. In the modified embodiment shown in FIG. 10B, the X-ray opaque portions 41 are provided discontinuously in the axial direction. In the modified embodiment shown in FIG. 10B, the gaps S provided between adjacent X-ray opaque portions 41 may be regularly provided or may be irregularly provided. The length of each of the gaps S, for example, is 1 to 10 mm.

FIG. 10C shows another modified embodiment of the wire-shaped visible part 4 shown in FIG. 2A of the embodiment. As shown in FIG. 10C, the X-ray opaque portions 41 made of an X-ray opaque material formed in a wire shape may have different densities in the axial direction. FIG. 10C shows an example in which, in the visible part 4, the density of the X-ray opaque portions 41 at both ends in the axial direction is higher than the density of the X-ray opaque portion 41 in the central part in the axial direction.

It should be noted that the configuration in which the density of the X-ray opaque portions 41 varies is not limited to the example of FIG. 10C, and for example, the density of the X-ray opaque portions 41 at both ends in the axial direction may be set lower than the density of the X-ray opaque portions 41 in the central part in the axial direction. In addition, the density of the X-ray opaque portion 41 on the distal side in the axial direction may be set higher than the density of the X-ray opaque portion 41 on the proximal side in the axial direction.

In addition to the above modified embodiments, for example, in the visible part 4, the X-ray opaque portion 41 may have a mesh shape, or the X-ray opaque portion 41 may have a tubular shape with a plurality of fine openings on the outer circumferential surface. In addition, the visible part 4 may include any appropriate combination of the embodiments and the above modified embodiments.

In the first and second embodiments, the delivery wire 3 made of a metal material functioning as the linear delivery member is described, the present invention is not limited thereto. The linear delivery member may be made of resin, or may be made of a composite material of metal and resin. In the first and second embodiments, the example in which the anchor device functions as a locking stent. However, the present invention is not limited thereto. The anchor device may be a balloon, for example.

In the second embodiment, as the first imaging method of the distal stabilizer 1, the example is described of continuously performing imaging in the first to third steps and operating the delivery wire 3 based on an X-ray transmission moving image that is imaged. In addition, as the second imaging method, the example is described of operating the delivery wire 3 based on an X-ray transmission image (a moving image or a still image) imaged in each of the first and second steps. However, the imaging method is not limited to these methods. A still image may be imaged in each step and the delivery wire 3 may be operated based on these still images. The operation of imaging a still image in each step may be repeatedly performed, and the delivery wire 3 may be operated based on the still images imaged in each step. In other words, it may be configured to perform the imaging in each step discontinuously and operate the delivery wire 3 based on the imaged X-ray transmission still images.

The imaging method may be a method including: a first step of imaging the visible part of the distal stabilizer sent together with the linear delivery member within a biological lumen; a second step of imaging the visible part during an operation of pushing or pulling the linear delivery member; and a third step of imaging the visible part after the operation of pushing or pulling the linear delivery member, in which the operations of imaging from the first step to third step are performed continuously or discontinuously.

### EXPLANATION OF REFERENCE NUMERALS

1 distal stabilizer
2 locking stent (anchor device)
3 delivery wire (linear delivery member)
4 visible part
5 first catheter
6 second catheter
10 delivery system
41 X-ray opaque portion
AN aneurysm
AN1 opening
TP target position
V biological lumen
V1 inner wall

## Claims

1. A distal stabilizer for use in catheter delivery within a biological lumen, the distal stabilizer comprising:
a linear delivery member;
an anchor device extending from a distal end of the linear delivery member and capable of being locked against an inner wall of the biological lumen; and
a visible part that is provided on a distal side of the linear delivery member and includes an X-ray opaque portion that is bendable in an axial direction together with the linear delivery member.

2. The distal stabilizer according to claim 1, wherein the visible part is provided along the axial direction from the distal end toward a proximal side of the linear delivery member.

3. The distal stabilizer according to claim 1 or 2, wherein the distal stabilizer is used in a biological lumen having a ratio of an estimated neck length of a lump formed within the biological lumen and a length in an axial direction of the visible part satisfying 1:1 to 1:20.

4. The distal stabilizer according to claim 1 or 2, wherein the length in the axial direction of the visible part is 10 mm to 500 mm.

5. The distal stabilizer according to claim 1 or 2, wherein the X-ray opaque portion is provided on 10% or more of a surface area of the visible part.

6. The distal stabilizer according to claim 1 or 2, wherein the X-ray opaque portion is configured by a tubular member provided on an outer circumferential surface of the linear delivery member.

7. The distal stabilizer according to claim 1 or 2, wherein the X-ray opaque portion is configured by a wire-shaped member wound around the outer circumferential surface of the linear delivery member.

8. The distal stabilizer according to claim 1 or 2, wherein the X-ray opaque portion is provided continuously or discontinuously in an axial direction of the visible part.

9. A method of imaging the distal stabilizer according to claim 1 within a biological lumen, the method comprising:
a first step of imaging the visible part of the distal stabilizer which is sent into a biological lumen together with the linear delivery member, prior to an operation of pushing or pulling the linear delivery member; and
a second step of imaging the visible part, after the operation of pushing or pulling the linear delivery member.
